# EUROPÄISCHE PATENTSCHRIFT

(11) **EP 0 367 163 B1**
(45) Veröffentlichungstag und Bekanntmachung des Hinweises auf die Patenterteilung: **03.01.1996**
(21) Anmeldenummer: 89120056.0
(22) Anmeldetag: 28.10.1989
(51) Int. Cl.: C12N 15/17, C12N 15/62

(54) **Verfahren zur Herstellung eines Insulin-Vorprodukts in Streptomyceten**
Process for producing an insulin precursor in streptomyces
Procédé de préparation d'un précurseur d'insuline chez les streptomyces

(30) Priorität: 03.11.1988 DE 3837273; 19.08.1989 DE 3927449
(43) Veröffentlichungstag der Anmeldung: 09.05.1990
(73) Patentinhaber: HOECHST AKTIENGESELLSCHAFT, D-65926 Frankfurt am Main (DE)
(72) Erfinder: Koller, Klaus-Peter, Dr., D-6232 Bad Soden am Taunus (DE); Riess, Günther, Johannes, Dr., D-6230 Frankfurt am Main 80 (DE); Uhlmann, Eugen, Dr., D-6246 Glashütten/Taunus (DE); Wallmeier, Holger, Dr., D-6232 Bad Soden am Taunus (DE)

(56) Entgegenhaltungen:
- EP-A- 0 161 629
- EP-A- 0 195 691
- EP-A- 0 281 090
- EP-A- 0 289 936
- WO-A-88/02005

## Beschreibung

Es wurde bereits ein Verfahren zur Herstellung von Fusionsproteinen vorgeschlagen, das dadurch gekennzeichnet ist, daß man das Strukturgen für das gewünschte Protein C-terminal an das gegebenenfalls modifizierte Tendamistatgen koppelt, diese Genstruktur in einer Streptomyceten-Wirtszelle zur Expression bringt und das sekretierte Fusionsprotein aus dem Kulturüberstand isoliert (DE 37 14 866 Al bzw. EP 0 289 936 A2). Die ältere Anmeldung betrifft weiterhin Genstrukturen, enthaltend das gegebenenfalls modifizierte Tendamistatgen, an das C-terminal ein Strukturgen für ein anderes Protein gekoppelt ist, Vektoren, die eine solche Genstruktur enthalten, Streptomycetenzellen, die einen solchen Vektor enthalten sowie Fusionsproteine, die durch einen N-ständigen Anteil von gegebenenfalls modifiziertem Tendamistat gekennzeichnet sind. Die ältere Anmeldung enthält Beispiele für Fusionsproteine, in denen die Aminosäuresequenz des Tendamistat über ein Brückenglied an die von Affen-Proinsulin gekoppelt ist.

In Weiterentwicklung dieses Erfindungsgedankens wurde nun gefunden, daß nach diesem Verfahren besonders gut ein Fusionsprotein hergestellt werden kann, in dem auf den Tendamistat-Anteil ein verkürztes Proinsulin folgt, dessen C-Kette lediglich aus einem oder zwei Lysin-Resten besteht. Diese Vorprodukte können besonders einfach und kostengünstig in Human-Insulin überführt werden.

Besondere Ausgestaltungen der Erfindung betreffen vorteilhafte Genstrukturen zur Amplifikation und Expression des Gens, das für das Fusions-Protein codiert. Weitere bevorzugte Ausgestaltungen der Erfindung werden im folgenden erläutert bzw. in den Patentansprüchen wiedergegeben.

Der erfindungsgemäß eingesetzten Genstruktur legt man vorteilhaft ein synthetisches Gen zugrunde, das für das verkürzte Proinsulinderivat codiert. Zweckmäßigerweise wird bei der Konstruktion dieses Gens auf den speziellen Codongebrauch von Streptomyceten Rücksicht genommen. Es hat sich gezeigt, daß hierdurch die Ausbeute an Fusionsprotein erhöht wird.

Vorteilhaft ist weiterhin der Einbau einer Terminatorsequenz in die synthetische Genstruktur, da auch hierdurch eine Steigerung der Syntheserate erzielt wird.

Ein großer Vorteil des erfindungsgemäßen Verfahrens besteht darin, daß die Fusionsproteine mit Hilfe des Plattentests nachgewiesen werden können, der in der EP-A1 0 161 629 in Beispiel 3 sowie in der deutschen Offenlegungsschrift 3 536 182, Beispiel 2, beschrieben ist. Hierdurch wird nicht nur die Selektion der interessierenden Klone, sondern auch die Aufarbeitung erheblich erleichtert, da der Einfluß der unterschiedlichen Parameter auf die Ausbeute leicht festgestellt werden kann.

Die erfindungsgemäß erhaltenen Fusionsproteine liegen offenbar in einer Konformation vor, die der des reifen Insulins entspricht oder zumindest nahe kommt. Hierdurch wird nicht nur die Weiterverarbeitung zum Insulin beträchtlich erleichtert, sondern es findet auch überraschenderweise bei Fermentationszeiten, die schon eine gute Ausbeute erbringen, kein merklicher Angriff durch die in das Fermentationsmedium ausgeschiedenen Proteasen statt.

Die erfindungsgemäße Modifikation des Proinsulinmoleküls mit seiner verkürzten C-Kette gestattet eine einfache Weiterverarbeitung zu Humaninsulin, nämlich durch chemische Spaltung mit Hydroxylamin und/oder durch enzymatische Spaltung mit Hilfe von Trypsin oder vorteilhaft Lysyl-Endoproteinasen. Die enzymatische Spaltung ist bevorzugt. Lysyl-Endoproteinasen spalten spezifisch carboxyterminal nach der Aminosäure Lysin. Durch die günstige Anordnung der A- und der B-Kette im erfindungsgemäßen Fusionsprotein entsteht durch Einwirkung der genannten Enzyme ein Insulinvorprodukt, in dem überraschenderweise die Disulfid-Brücken richtig geknüpft sind.

Zweckmäßigerweise sieht man in der Genkonstruktion zwischen dem Tendamistat-Anteil und dem Beginn des Proinsulinmoleküls ein Brückenglied vor, das die Abspaltung des Proinsulin-Derivats vom Tendamistat-Anteil mit dem gleichen Enzym zuläßt, das zur Aufspaltung des Proinsulin-Derivats in die beiden Insulin-Ketten dient.

Spaltet man die erfindungsgemäßen Fusionsproteine mit einer Lysyl-Endopeptidase, so erhält man - je nach der Konstruktion der modifizierten C-Kette - Des-B³°-Insulin, das durch Transpeptidierung in Humaninsulin umgewandelt werden kann, oder B³¹-Lys-Insulin bzw. B³¹-Lys-B³-Lys-Insulin, welche beispielsweise durch den Einsatz von Carboxypeptidase B zu Humaninsulin umgewandelt werden können.

Besonders hohe Ausbeuten an dem gewünschten Protein erhält man, wenn man Genkonstruktionen mit einem verkürzten Tendamistatgen einsetzt. Diese erfindungsgemäße Ausgestaltung des Verfahrens hat den großen Vorteil, daß der Anteil des modifizierten Insulins im Fusionsprotein etwa die Hälfte ausmacht, also wesentlich weniger "Ballast" enthält. Die korrekte Faltung des Fusionsproteins wird durch die Verkürzung des Tendamistat-Anteils nicht beeinträchtigt, so daß also auch mit dem erfindungsgemäßen kleineren Fusionsprotein die vorteilhafte Aufarbeitung möglich ist. Dieser Vorteil muß auch nicht durch eine erhöhte Abbauungsrate durch die wirtseigenen Proteasen erkauft werden - unerwarteterweise ergab sich sogar eine erhöhte Stabilität gegenüber diesen Proteasen.

Die Erfindung erlaubt somit eine ganze Reihe von vorteilhaften Genkonstruktionen, die zu Insulin-Vorprodukten führen, die leicht vom "Ballastanteil" des Fusionsproteins abgetrennt werden können. Durch diese einfache Aufarbeitung wird die Ausbeute an Humaninsulin zusätzlich verbessert.

Die Abtrennung des Fusionsproteins aus dem Kulturmedium, seine Weiterverarbeitung zur Insulin-Vorstufe und deren Umwandlung in Humaninsulin können nach an sich bekannten Methoden erfolgen. So läßt sich das Fusionsprotein vorteilhaft durch Adsorptions- oder Ionenaustauschchromatographie und/oder Gelfiltration isolieren und der Proinsulin-Anteil chemisch oder vorteilhaft enzymatisch abspalten. Die Konstruktion entsprechender Brückenglieder ist allgemein bekannt und beispielsweise in der EP-A2 0 229 998 beschrieben.

Aus der EP-B 0 089 007 sind Analoge von Präpro- und Proinsulin bekannt, die am C-Ende der Präkette (bzw. am N-Terminus des Proinsulins) Lys oder Arg tragen (was auch bei den erfindungsgemäßen Konstruktionen bevorzugt ist), deren B-Kette mit B⁹-Lys endet und wobei das C-Peptid auf Lys oder Arg verkürzt sein kann, so daß also in der Proinsulinstruktur auf B⁹-Lys im einfachsten Fall nur Lys oder Arg folgt, woran sich die A-Kette anschließt. Diese Verbindungen dienen als Vorprodukte für die Herstellung von Insulinen mit Hilfe von Trypsin oder trypsinähnlichen Endopeptidasen und einem Ester einer natürlichen Aminosäure, die gegebenenfalls Schutzgruppen trägt.

Insulin-Vorläufer, bei denen die B- und A-Kette durch das Brückenglied -X-Y- verbunden sind, in dem X und Y gleich oder verschieden sind und für Lys und Arg stehen, sind aus der EP-A 0 195 691 bekannt. Diese Insulin-Vorläufer werden aus Hefe exprimiert und dann durch enzymatische Umwandlung in Humaninsulin überführt. Insulin-Vorläufer mit verkürzter C-Kette sind auch aus der EP-A 0 163 529 bekannt. In den EP-B 0 132 769 und 0 132 770 sind Insulin-Derivate sowie diese enthaltende pharmazeutische Mittel beschrieben.

In den folgenden Beispielen wird die Erfindung näher erläutert. Prozentangaben beziehen sich auf das Gewicht, sofern keine anderen Angaben gemacht sind.

Die Figur erläutert die erfindungsgemäße Genkonstruktion des Beispiels 1. Sie ist nicht maßstabsgerecht.

### Beispiel 1

Das in der Tabelle 1 wiedergegebene synthetische Gen (1) wird in an sich bekannter Weise nach der Phosphoamidit-Methode chemisch synthetisiert. Für die Codon-Auswahl wurde die Präferenz der Streptomyceten für G und C berücksichtigt. Wie bei dem für Affen-Proinsulin codierenden Gen der älteren Anmeldung (dort Tabelle 2) weist auch das Gen (1) gemäß Tabelle 1 am 5'-Ende eine für das Restriktionsenzym EcoRI typische überhängende Sequenz auf. Nach dem Strukturgen finden sich zwei Stopp-Codons und eine Linkersequenz mit der Erkennungsstelle für das Enzym SalI. Am 3'-Ende findet sich die überhängende Sequenz entsprechend dem Restriktionsenzym HindIII.

Das handelsübliche Plasmid pUC19 wird mit den Enzymen EcoRI und HindIII geschnitten und das synthetische Gen (1) gemäß Tabelle 1 hineinligiert. Man erhält so das Plasmid PI1 (2). Nach Amplifikation wird das synthetische Gen mit den Enzymen EcoRI und SalI als Fragment (3) herausgeschnitten und für die weiter unten beschriebene Konstruktion eingesetzt.

Das Plasmid pUC19 wird mit SmaI komplett verdaut und mit der in der Tabelle 2 wiedergegebenen Terminatorsequenz (4) ligiert. Plasmide, die diese Sequenz in der korrekten Orientierung enthalten, erhalten die Bezeichnung pT1 (5). Dieses Plasmid (5) wird mit EcoRI geöffnet und die Schnittstelle mit DNA-Polymerase (Klenow-Fragment) aufgefüllt. Durch Religieren erhält man das Plasmid pT2 (6). Dieses Plasmid wird mit den Enzymen SalI und SphI geöffnet und das große Fragment (7) isoliert.

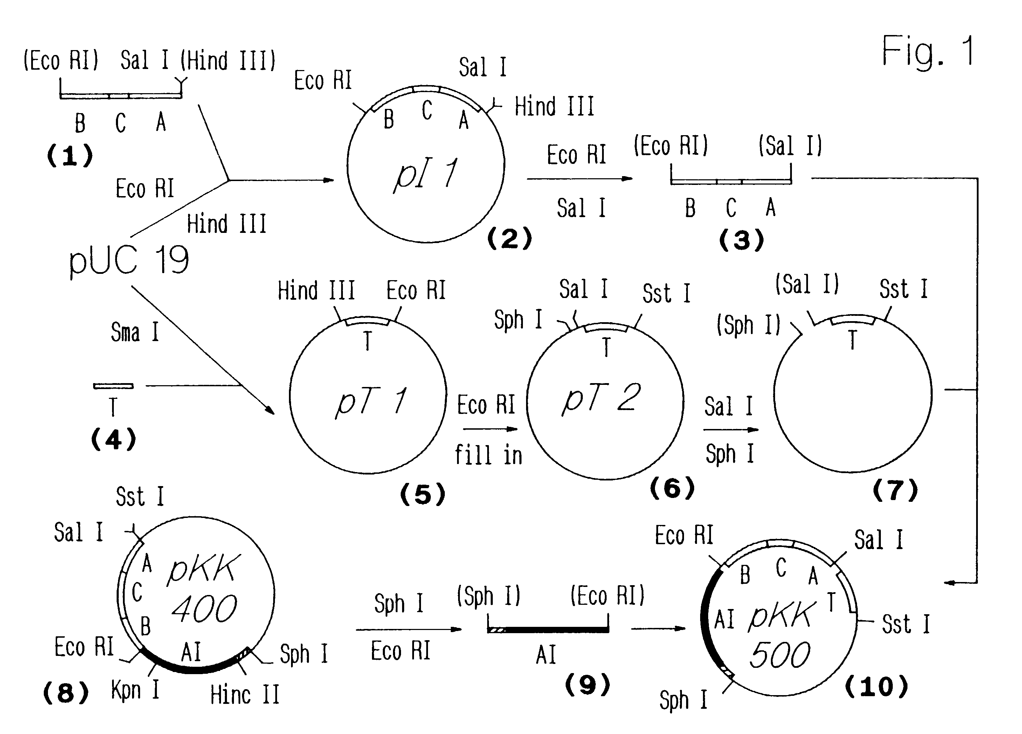

Das Plasmid pKK400 (8) (vgl. ältere Anmeldung, Figur 4, (20)) wird mit SphI und EcoRI geschnitten und das kleine Fragment (9) mit dem Tendamistatgen isoliert.

Durch Ligation der Fragmente (3), (7) und (9) erhält man das Plasmid pKK500 (10), in dem auf die Tendamistat-Sequenz das für 12 Aminosäuren codierende Brückenglied 
Phe Asn Ala Met Ala Thr Gly Asn Ser Asn Gly Lys
TTC AAT GCG ATG GCC ACC GGG ATT TCG AAC GGC AAG
AAG TTA CGC TAC CGG TGG CCC TAA AGC TTG CCG TTC
EcoRI
 und hierauf das Gen für das erfindungsgemäß modifizierte Proinsulin folgen. Die korrekte Anordnung wird durch Schneiden mit SphI und SstI kontrolliert, wobei aus dem ca. 3,5 kb großen Plasmid ein Fragment von 833 bp erhalten wird. Durch DNA-Sequenzierung nach der Di-desoxy-Methode wird die Richtigkeit der Sequenz bestätigt.

Erfindungsgemäße Genkonstruktionen, in denen das als C-Peptid fungierende Lys durch ein weiteres Lys ergänzt wird, werden analog hergestellt. Es wird hierzu das für Lys codierende Triplett AAG verdoppelt. Man erhält analog das Plasmid pI2 und daraus den Vektor pKK600.

### Beispiel 2

Analog dem in der älteren Anmeldung vorgeschlagenen Vektor pGF1 werden aus den Vektoren pKK500 und pKK600 die Expressions-Plasmide pGF2 und pGF3 hergestellt. Hierzu werden aus den Vektoren pKK500 und pKK600 durch Doppelverdauung mit SphI und SstI jeweils das Insert von 823 bzw. 826 bp isoliert und diese DNA-Fragmente in das mit den gleichen Enzymen gespaltene Expressions-Plasmid pIJ702 ligiert. Das Ligationsgemisch wird in S. lividans TK 24 transformiert und aus Thiostrepton-resistenten Transformanten, die Tendamistat-Aktivität zeigen (Plattentest), die Plasmid-DNA isoliert. Alle positiven Klone enthalten das eingesetzte Insert aus pKK500 bzw. pKK600.

Die Expression des codierten Fusionsproteins kann in bekannter Weise erfolgen. Inkubiert man den transformierten Stamm S. lividans TK 24 vier Tage bei 28°C im Schüttelkolben und trennt das Mycel von der Kulturlösung durch Zentrifugieren ab, so kann man das Fusionsprotein in der klaren Lösung wie folgt nachweisen:

10 bis 100 µl Lösung werden mit 20 bis 200 µl 15 %iger Trichloressigsäure versetzt, das ausgefallene Protein wird durch Zentrifugieren angereichert, gewaschen und in SDS-haltigem Probenpuffer (U. Laemmli, Nature 227 (1970) 680-685) aufgenommen. Nach 2 Minuten Inkubation bei 90°C trennt man elektrophoretisch auf einem 10-17 %igen SDS-Polyacrylamidgel auf. Man erhält ein Protein vom Molekulargewicht 15 kD, also im erwarteten Molekulargewichtsbereich für das Fusionsprotein aus Tendamistat und Proinsulin. Das Fusionsprotein reagiert sowohl mit Antikörpern gegen Tendamistat als auch mit Antikörpern gegen Insulin.

### Beispiel 3

Der Expressionsvektor pTF2 (DE 37 14 866 Al, Beispiel 4) wird mit den Restriktionsenzymen EcoRI und SstI verdaut und das Fragment, das Affen-Proinsulin codiert, entfernt. Das 5,65 kbp große Fragment wird für die unten beschriebene Ligierungsreaktion verwendet.

Aus dem Plasmid pKK500 (Beispiel 1) wird mit denselben Restriktionsenzymen ein DNA-Fragment der Größe 285 bp ausgeschnitten, das das verkürzte Proinsulin-Gen sowie die Terminationssequenz enthält.

Die Ligation des 5,65 kbp großen Fragments aus pTF2 mit dem 285 bp großen Fragment aus pKK500 ergibt das Expressionsplasmid pTF3.

Werden Protoplasten von Streptomyces lividans TK24 mit dem Ligationsgemisch transformiert, so entstehen Klone, die Thiostrepton-resistent sind und ein Fusionsprotein sekretieren, das mit Antikörpern gegen Proinsulin reagiert. Dieses Fusionsprotein besteht aus den ersten 41 Aminosäuren von Tendamistat, dem Brückenglied Pro-Ser-Leu-Asn-Ser-Asn-Gly-Lys und dem verkürzten Proinsulin.

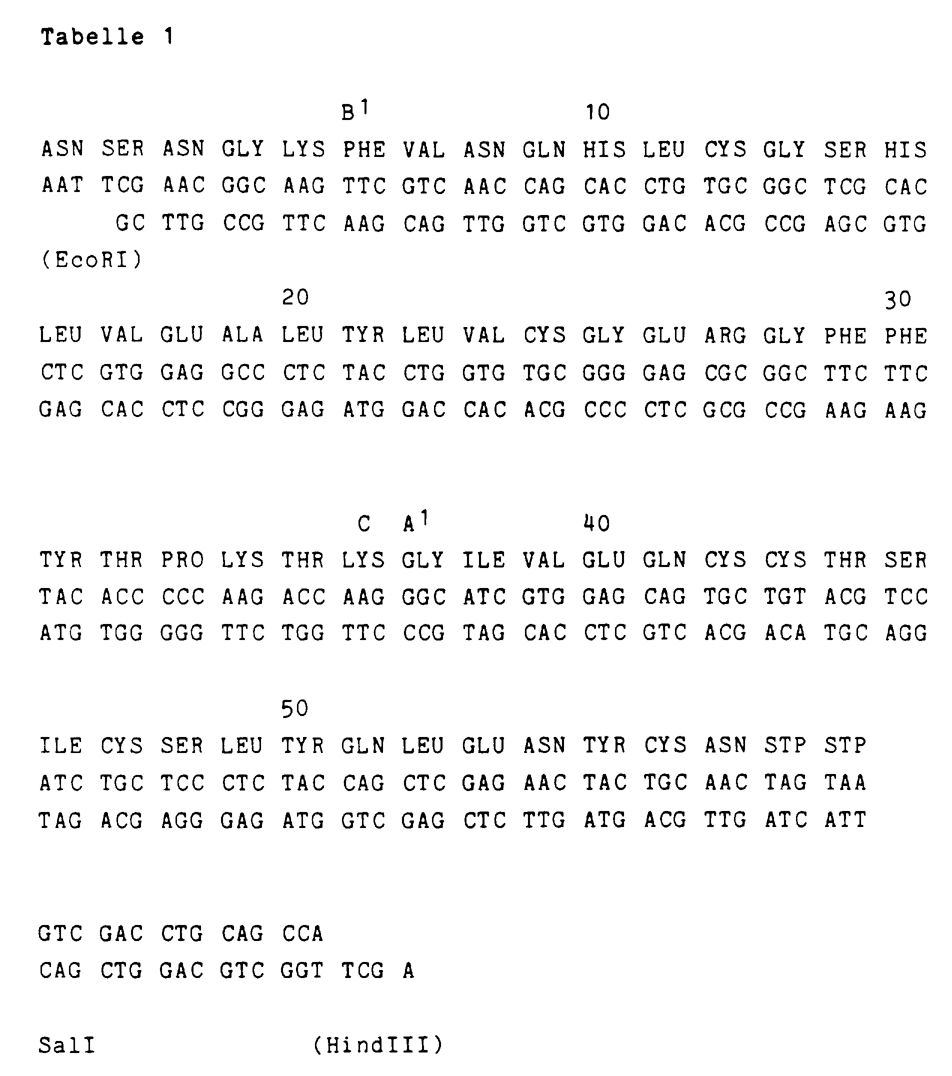

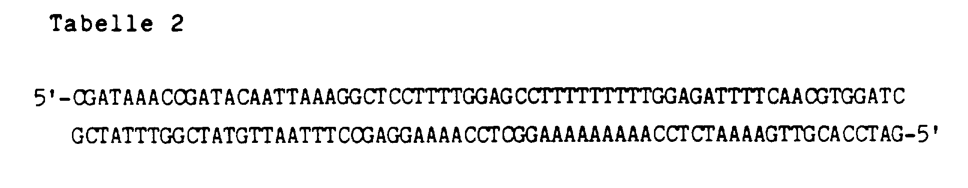

## Patentansprüche (Patentansprüche für folgende(n) Vertragsstaat(en): AT, BE, CH, DE, FR, GB, GR, IT, LI, LU, NL, SE)

1. Verfahren zur Herstellung von Fusionsproteinen durch Koppeln des Strukturgens für das gewünschte Protein an den C-Terminus des gegebenenfalls modifizierten Tendamistatgens, Expression dieser Genstruktur in einer Streptomyceten-Wirtszelle und Isolieren des sekretierten Fusionsproteins aus dem Überstand, dadurch gekennzeichnet, daß das Strukturgen für das gewünschte Protein für ein Proinsulin-Derivat codiert, bei dem die B-Kette über ein Brückenglied an die A-Kette gebunden ist, das für Lys oder Lys-Lys codiert.

2. Genstruktur, enthaltend das gegebenenfalls modifizierte Tendamistatgen, an das C-terminal das in Anspruch 1 definierte Strukturgen gekoppelt ist.

3. Vektor, enthaltend eine Genstruktur nach Anspruch 2.

4. Streptomycetenzelle, enthaltend einen Vektor nach Anspruch 3.

5. Fusionsprotein, gekennzeichnet durch einen N-ständigen Anteil von gegebenenfalls modifiziertem Tendamistat, an dessen C-Terminus das in Anspruch 1 definierte Proinsulin gebunden ist.

## Patentansprüche (Patentansprüche für folgende(n) Vertragsstaat(en): ES)

1. Verfahren zur Herstellung von Fusionsproteinen durch Koppeln des Strukturgens für das gewünschte Protein an den C-Terminus des gegebenenfalls modifizierten Tendamistatgens, Expression dieser Genstruktur in einer Streptomyceten-Wirtszelle und Isolieren des sekretierten Fusionsproteins aus dem Überstand, dadurch gekennzeichnet, daß das Strukturgen für das gewünschte Protein für ein Proinsulin-Derivat codiert, bei dem die B-Kette über ein Brückenglied an die A-Kette gebunden ist, das für Lys oder Lys-Lys codiert.

## Claims (Claims for the following Contracting State(s): AT, BE, CH, DE, FR, GB, GR, IT, LI, LU, NL, SE)

1. A process for the preparation of fusion proteins by coupling the structural gene for the desired protein to the C terminus of the optionally modified tendamistat gene, expression of this gene structure in a Streptomycetes host cell and isolation of the secreted fusion protein from the supernatant, wherein the structural gene for the desired protein codes for a proinsulin derivative in which the B chain is linked to the A chain via a bridging member which codes for Lys or Lys-Lys.

2. A gene structure containing the optionally modified tendamistat gene to which the structural gene defined in claim 1 is coupled C-terminally.

3. A vector containing a gene structure as claimed in claim 2.

4. A Streptomycetes cell containing a vector as claimed in claim 3.

5. A fusion protein which has an N-terminal portion of optionally modified tendamistat to whose C terminus the proinsulin defined in claim 1 is linked.

## Claims (Claims for the following Contracting State(s): ES)

1. A process for the preparation of fusion proteins by coupling the structural gene for the desired protein to the C terminus of the optionally modified tendamistat gene, expression of this gene structure in a Streptomycetes host cell and isolation of the secreted fusion protein from the supernatant, wherein the structural gene for the desired protein codes for a proinsulin derivative in which the B chain is linked to the A chain via a bridging member which codes for Lys or Lys-Lys.

## Revendications (Revendications pour l'(les) Etat(s) contractant(s) suivant(s): AT, BE, CH, DE, FR, GB, GR, IT, LI, LU, NL, SE)

1. Procédé pour la production de protéines de fusion, par couplage du gène structural, codant pour la protéine recherchée, à l'extrémité C-terminale du gène éventuellement modifié du tendamistat, expression de cette structure génique dans une cellule hôte de *Streptomyces* et isolement de la protéine sécrétée à partir du surnageant, caractérisé en ce que le gène structural codant pour la protéine recherchée code pour un dérivé de proinsuline dans lequel la chaîne B est liée à la chaîne A par un chaînon pontant qui consiste en Lys ou Lys-Lys.

2. Structure génique, contenant le gène éventuellement modifié du tendamistat, à l'extrémité C-terminale duquel est couplé le gène structural défini dans la revendication 1.

3. Vecteur, contenant un gène structural selon la revendication 2.

4. Cellule de *Streptomyces,* contenant un vecteur selon la revendication 3.

5. Protéine de fusion, caractérisée par un segment N-terminal de tendamistat éventuellement modifié, à l'extrémité C-terminale duquel est liée la proinsuline définie dans la revendication 1.

## Revendications (Revendications pour l'(les) Etat(s) contractant(s) suivant(s): ES)

1. Procédé pour la production de protéines de fusion, par couplage du gène structural, codant pour la protéine recherchée, à l'extrémité C-terminale du gène éventuellement modifié du tendamistat, expression de cette structure génique dans une cellule hôte de *Streptomyces* et isolement de la protéine sécrétée à partir du surnageant, caractérisé en ce que le gène structural codant pour la protéine recherchée code pour un dérivé de proinsuline dans lequel la chaîne B est liée à la chaîne A par un chaînon pontant qui consiste en Lys ou Lys-Lys.
